# EUROPÄISCHE PATENTSCHRIFT

(11) **EP 3 068 447 B1**
(45) Veröffentlichungstag und Bekanntmachung des Hinweises auf die Patenterteilung: **06.03.2019**
(21) Anmeldenummer: 14809300.8
(22) Anmeldetag: 12.11.2014
(51) Int. Cl.: A61L 15/20, A61L 15/26, A61L 15/40, A61L 15/44, A61L 15/46, A61L 26/00

(54) **FLUID ZUR WUNDBEHANDLUNG UND WUND- ODER HAUTAUFLAGE**
WOUND HEALING FLUID AND WOUND OR SKIN DRESSING
FLUIDE DE TRAITEMENT DES PLAIES ET PANSEMENT POUR LES PLAIES OU LA PEAU

(30) Priorität: 12.11.2013 DE 102013018941
(43) Veröffentlichungstag der Anmeldung: 21.09.2016
(73) Patentinhaber: Tosama sanitetnega materiala d.o.o., 1230 Domzale (SI)
(72) Erfinder: CERLINI, Jasna, 1000 Ljubljana (SI); KRALJ KUNCIC, Marjetka, 4275 Begunje na Gorenjskem (SI); SIMNIC SOLINC, Mojca, 1131 Ljubljana (SI); ZABRET, Andrej, 1218 Komenda (SI); LUKAN CIVIC, Masa, 1236 Trzin (SI); CVJETICANIN, Mladen, 1000 Ljubljana (SI)
(74) Vertreter: Marton, Dan-Robert
(86) Internationale Anmeldenummer: PCT/EP2014/074297
(87) Internationale Veröffentlichungsnummer: WO 2015/071274

(56) Entgegenhaltungen:
- WO-A1-2004/093569
- WO-A1-2012/134770
- WO-A2-2009/124578
- GB-A- 2 048 070
- US-A1- 2004 131 693
- US-B1- 6 171 604
- European Pharmacopoeia 6.0, Seiten 1670-1671, Eintrag: Dexpanthenol.
- Stryer: "Biochemie 4. Auflage", 1996, Spektrum Akademischer Verlag, Heidelberg page 477,
- Eteraf-Oskouei, Najafi: "Traditional and modern uses of natural honey in human diseases: a review", Iranian journal of basic medical sciences, June 2013 (2013-06), pages 731-742, XP055320724, Iran Retrieved from the Internet: URL:http://ijbms.mums.ac.ir/article_988_bd f33cc2c4a3335246dee5766339d346.pdf
- SIMON ET AL.: "Medical Honey for Wound Care-Still the 'Latest Resort'?", EVIDENCE-BASED COMPLEMENTARY AND ALTERNATIVE MEDICINE, vol. 6, no. 2, 2009, pages 165-173, XP055100621, ISSN: 1741-427X, DOI: 10.1093/ecam/nem175
- EBNER ET AL.: "Topical use of dexpanthenol in skin disorders", AM. J. CLIN. DERMATOL, vol. 3, no. 6, 2002, pages 427-433, XP009181863, ISSN: 1175-0561, DOI: 10.2165/00128071-200203060-00005

## Beschreibung

### Hintergrund der Erfindung

Die vorliegende Erfindung betrifft ein Fluid zur Behandlung von Wunden und ferner eine Wund- oder Hautauflage.

### Stand der Technik

Wundauflagen sollen eine Reihe verschiedener Anforderungen erfüllen, um die Wundheilung zu unterstützen. Dazu können diese Auflagen mit einer Zusammensetzung, wie beispielsweise einer Salbe, versehen werden, welches die Heilung der Wunde unterstützen kann. Erstens soll die Wunde vor Umwelteinflüssen, wie z.B. Eindringen von Fremdkörpern und Einwirkung von äußerem Druck, geschützt werden. Zweitens sollte diese aber auch keimfrei und ggf. sterilisierbar sein und damit die Wahrscheinlichkeit einer Nebeninfektion zu senken. Ferner dienen die Auflagen dazu, die Wunde warm und feucht zu halten, da Wunden besser heilen, wenn sie unter Okklusion feucht gehalten werden. Als Ursache für die "heilende" Wirkung eines feuchten Milieus wird in der Literatur unter anderem folgende Wirkungsweise angeführt: Durch Sauerstoffausschluss ist die Wunde gezwungen, über das Blut Sauerstoff in das Wundgebiet zu bringen. Dies erfolgt durch vermehrte Gefäßeinsprossung unter Gefäßneubildung und damit geförderte Wundheilung. Dieses kann ergänzend vom eingesetzten Fluid in der Wundauflage unterstützt werden. Andererseits soll die Wundauflage eine gute Saugfähigkeit und eine ausreichende Saugkapazität aufweisen, um ein Abziehen des Wundsekretes zu ermöglichen und die Bildung von feuchten Kammern zu vermeiden. Dies wird durch die verwendeten Schichten der Auflage gewährleistet.

WO 2012/134770 A1 beschreibt eine Zusammensetzung, die eine Kombination von Polymer-Komponenten und Honig enthält. Die Zusammensetzung kann in medizinischen Produkten verwendet werden. WO 2004/093569 A1 betrifft eine topische Zusammensetzung auf der Basis von Honig.

Eine Wundauflage soll außerdem eine ausreichende Nassfestigkeit aufweisen und das Zurückbleiben von Rückständen verhindern, welche die Wundheilung irritieren könnten. Um den Prozess der Wundheilung zu fördern, sollte eine Wundauflage eine ausreichende Sauerstoff- und Wasserdampfdurchlässigkeit und damit Atmungsaktivität gewährleisten.

Sie soll sich der Wunde anschmiegen, um eine gleichmäßige Aufnahme des Wundsekrets bzw. gegebenenfalls Abgabe von Wirk- Stoffen zu ermöglichen, hautfreundlich sowie leicht zu applizieren und zu entfernen sein, um die Wundruhe zu gewährleisten.

Eine solche Wundauflage ist beispielsweise aus der WO 2009/124578 A2 bekannt, in welcher eine Wundauflage mit einer inkorporierten Salbe offenbart ist. Diese Wundauflage gemäß dem Stand der Technik umfasst ein wasserdampfdurchlässiges Trägermaterial, eine undurchlässige Barriereschicht und weiter eine Textilbasis. In der Textilbasis soll die Salbe gemäß der WO 2009/124578 A2 inkorporiert werden. Folglich kann ein feuchtes Wundheilungsmilieu gewährleistet werden.

### Der Erfindung zugrundeliegende Aufgabe

Der Erfindung liegt die Aufgabe zugrunde, ein Fluid und eine Auflage zur Behandlung von Wunden bereitzustellen, welche eine möglichst schmerzfreie oder schmerzlindernde Anwendung gewährleisten.

### Erfindungsgemäße Lösung

Die Lösung der gestellten Aufgabe gelingt durch ein Fluid mit den Merkmalen des Anspruchs 1 und mit einer Auflage mit den Merkmalen des Anspruchs 9. Vorteilhafte Aus- und Weiterbildungen, welche einzeln oder in Kombination miteinander eingesetzt werden können, sind Gegenstand der abhängigen Ansprüche.

Das erfindungsgemäße Fluid weist folgende Zusammensetzung auf: medizinischer Honig, ein Lokalanästhetikum, Dexpanthenol und ein Wirkstoffträger. Das Fluid kann vorteilhaft bei der Versorgung von Wunden, insbesondere von offenen Wunden, verwendet werden. Weiter durch die erfindungsgemäße Zusammensetzung kann vorteilhaft eine schnell einwirkende Schmerzlinderung beim Auftragen des Fluids auf eine Wunde gewährleistet werden.

Der medizinische Honig weist eine hohe osmotische Aktivität auf und folglich kann die vorteilhaft Wundheilung angeregt werde. Der medizinische Honig wirkt auf die häufigsten in einer Wunde auftretenden Mikroorganismen bakteriostatisch, so dass die Heilung schneller erfolgen kann. Weiter bildet der medizinische Honig eine Gelartige Schicht in Reaktion auf das in der Wunde entstehende Exsudat und schafft somit feuchte Umgebung (feuchtes Milieu) zur Wundheilung. Diese Schicht überdeckt die Wunde und kann beispielsweise bei einer Verwendung einer Hautauflage den direkten Kontakt zwischen der offenen Wunde und der Auflage minimieren. Somit kann diese Gelartige Schicht als Schutzschicht dienen.

Durch den Einsatz eines Lokalanästhetikums gemäß der erfindungsmäßigen Zusammensetzung kann im Wesentlichen eine Schmerzlinderung bei einer Anwendung des Fluids gewährleistet werden. Einerseits kann beim Auftragen des Fluids auf eine Wunde der Schmerz, der durch das Auftragen entsteht, gelindert werden und andererseits kann der Schmerz, der durch die osmotische Wirkung des Honigs auftritt, ebenfalls gelindert werden. Das Lokalanästhetikum kann folglich direkt im Wundbereich, nämlich lokal, wirken.

Dexpanthenol (oder Pantothensäure) kann ebenfalls direkt im Wundbereich auf die Epithelzellen einwirken, und dient im Wesentlichen als Feuchtigkeitsspender, der die Hornschicht der Haut positiv beeinflusst. Die Haut bleibt folglich elastisch und weich und erhält, die im Wundbereich notwendige Pflege. Dexpanthenol ist auch als (Pro-) Vitamin B5 bekannt. Dexpanthenol ist die D-Form von Panthenol. Dexpanthenol kann im Körper in Pantothensäure umgewandelt werden. Dieses Vitamin ist wichtig für eine normale epitheliale Funktion, die Hautfeuchtigkeit, den Zustand des Stratum corneums, sowie für die Hautelastizität. Insbesondere kann Dexpanthenol gemäß der European Pharmacopoeia, insbesondere gemäß European Pharmacopoeia 6.0, Abschnitt "Dexpanthenol" verwendet werden.

Weiter sieht die Zusammensetzung des erfindungsgemäßen Fluids die Zugabe eines Wirkstoffträgers, der im Wesentlichen als Lösungsmittel für das Lokalanästhetikum dient. Der Wirkstoffträger kann aber auch so dosiert werden, dass beispielsweise weitere Substanzen dem erfindungsgemäßen Fluid beigemischt werden können.

Gemäß eines weiteren Aspekts der vorliegenden Erfindung wird eine Wund oder Hautauflage bereitgestellt. Die Auflage weist mindestens eine erste mit einem Fluid zur Behandlung von Wunden versetzte Trägerschicht. Die Trägerschicht kann synthetisch oder aus einem natürlichen Material ausgebildet sein. Bevorzugt ist die Wundauflage gemäß der Erfindung dergestalt ausgebildet, dass diese (Trägerschicht und/oder Wund-oder Hautauflage) komplett mit dem Fluid getränkt ist und in der Anwendung direkt auf die Hautwunde appliziert bzw. aufgetragen werden kann. Die Wundauflage gemäß der Erfindung weist mindestens eine erste Trägerschicht auf die mit dem Fluid zur Wundbehandlung getränkt ist. Es ist jedoch vorstellbar, dass weitere Schichten vorgesehen sind die direkt oder indirekt mit dem Fluid versetzt werden. So kann beispielsweise vorstellbar sein, dass eine erste Schicht, insbesondere eine erste synthetische Schicht vorgesehen ist, und eine weitere Schicht, insbesondere eine weitere Schicht aus einem natürlichen Material, daran angeordnet ist. Diese Schichten können beispielsweise übereinander auf die Wunde aufgelegt werden. Eine dieser beiden Schichten kann direkt mit dem Fluid versetzt werden, und anschließend durch Kapillareffekte wird die weitere Schicht mit dem Fluid indirekt versetzt.

Durch die erfindungsgemäße Wundauflage wird folglich ein feuchtes Milieu zur Wundheilung bereitgestellt und weiter kann das verwendete Fluid eine schmerzlindernde Wirkung bei der Anwendung auf der Haut entfalten.

Durch die erfindungsgemäße Wundauflage kann folglich eine feuchte Umgebung im Wundbereich gewährleistet werden, welche weiter durch den medizinischen Honig eine osmotische Aktivität zur Wundheilung gewährleistet. Das Lokalanästhetikum in der Zusammensetzung des Fluids erlaubt ferner eine im Wesentlichen schmerzfreie Anwendung und weiter kann eine schmerzlindernde Wirkung während der gesamten Anwendungszeit bereitgestellt werden. Erfindungsgemäße Haut- oder Wundauflagen und Fluide verringern zudem die Gefahr einer Wundinfektion, verringern unangenehme Gerüche, stimulieren die Säuberung von nekrotischem Gewebe (insbesondere aufgrund der osmotischen Aktivität von Fluidbestandteilen), stimulieren Granulation und Epithelisierung, fördern eine Wundheilung ohne Narbenbildung, und haften nicht fest an der Wunde an.

### Bevorzugte Ausgestaltung der Erfindung:

In einer bevorzugten Ausführung der Erfindung ist der medizinische Honig durch einen auf Kastanien basierenden Honig gebildet. Der medizinische Honig sollte vorzugsweise stets eine vorherbestimmte geographische Herkunft aufweisen. Ferner sollte dieser vorzugsweise eine bestimmte Viskosität und/oder Konsistenz aufweisen, so dass nicht immer klinische Studien wiederholt werden müssen. So können unterschiedliche Chargen verwendet werden, welche alle dieselben vorherbestimmten Eigenschaften aufweisen. Als besonders heilend und somit vorteilhaft hat sich die Verwendung eines auf Kastanien basierenden medizinischen Honigs herausgestellt. Die in ihm enthaltenen Inhaltsstoffe können die osmotische Wundheilung besonders fördern und die Anwendung hat sich als problemlos und zugleich heilend herausgestellt.

Gemäß einer Ausführungsform der vorliegenden Erfindung kann Benzocain als Lokalanästhetikum zum Einsatz kommen. Benzocain wirkt schnell auf die betreffende Stelle und der Benutzer des Fluids empfindet folglich keinen Schmerz bei der Verwendung. Klinische Studien haben gezeigt, dass Benzocain in Verbindung mit den weiteren Zusätzen des Fluids besonders schmerzlindernd wirkt. Alternativ kann Diclofenac eingesetzt werden, welches ebenfalls zu guten Ergebnissen bei der Schmerzlinderung führen kann.

Der medizinische Honig ist zu einem Anteil von 87 Gew.-% bis 88 Gew.-%, insbesondere 87,3 Gew.-% bis 87,6 Gew.-%, bevorzugt 87,5 Gew.-%, bezogen auf die Gesamtfluidmasse umfasst. Diese Konzentrationen haben eine besonders heilende Wirkung bei betroffenen Hautpartien gezeigt. Ferner kann das Gesamtfluid einfach gehandhabt werden, und weist folglich immer die gewünschte Viskosität bzw. Konsistenz auf.

Lokalanästhetikum ist zu einem Anteil von 2 Gew.-% bis 3 Gew.-%, insbesondere 2,4 Gew.-% bis 2,6 Gew.-%, bevorzugt 2,5 Gew.-%, bezogen auf die Gesamtfluidmasse umfasst. Diese Konzentrationen im Zusammenspiel mit den Weiteren Komponenten des Fluids, haben sich als besonders vorteilhaft herausgestellt. So konnte bestätigt werden, dass diese Konzentration eine sehr schnelle lindernde Wirkung während der Anwendung entfalten konnte. Insbesondere der Anteil der 2,5 Gewichtsprozent (Gew.-%) beträgt, hat eine besonders schnelle Schmerzlinderung gezeigt, und das verwendete Lokalanästhetikum (insbesondere Benzocain) konnte schnell die Nerven der betroffenen Hautpartie lokal betäuben, so dass die Schmerzlinderung sofort eintreten kann. Dies wird von der anwendenden Person als sehr angenehm und wohltuend empfunden.

Dexpanthenol ist zu einem Anteil von 2 Gew.-% bis 7 Gew.-%, insbesondere 4,5 Gew.-% bis 5,5 Gew.-%, bevorzugt 5 Gew.-%, bezogen auf die Gesamtfluidmasse umfasst. Diese Dosierung hat insbesondere mit der Honigzusammensetzung eine sehr gute pflegende Wirkung offenbart. Insbesondere 5 Gew.-% haben eine deutliche Verbesserung der Hautelastizität im Zusammenhang mit der osmotischen Wirkung des medizinischen Honigs gezeigt. Nach der Anwendung konnte eine sehr gute hautpflegende Wirkung der oben erwähnten Konzentration beobachtet werden.

Es ist bevorzugt, dass der Wirkstoffträger zu einem Anteil von 4 Gew.-% bis 6 Gew.-%, insbesondere 4,9 Gew.-% bis 5,1 Gew-%, bevorzugt 5 Gew.-%, bezogen auf die Gesamtfluidmasse umfasst ist. Der Wirkstoffträger nimmt in dieser Konzentration am besten die wirkenden Additive des erfindungsgemäßen Fluids auf. Insbesondere bei einem Wert von 5 Gew.-% konnte das Lokalanästhetikum sehr schnell in der Wirkstoffträger vermischt werden, welches sehr vorteilhaft während der Herstellung des Fluids ist. So hat diese Dosierung die Produktionszeit des erfindungsgemäßen Fluids deutlich verkleinert, welches einem kosteneffektiven Produkt entsprechen kann.

In vorteilhafter Weise kann als Wirkstoffträger Macrogol bzw. Polyethylenglycol PEG eingesetzt werden. Macrogol hat sich im Zusammenspiel mit dem Lokalanästhetikum, insbesondere Benzocain, als sehr gut verträglich herausgestellt. Weiter erhöht Macrogol die Lagerzeit des erfindungsgemäßen Fluids, so dass dieses länger verwendet werden kann.

Fluide können insbesondere den medizinischen Honig und das Lokalanästhetikum (insbesondere Benzocain) in einem Gewichtsverhältnis (medizinischer Honig : Lokalanästhetikum) von 25 :1 bis 45 : 1, vorzugsweise 30 : 1 bis 40 : 1, weiter vorzugsweise von 34 : 1 bis 36 : 1, besonders bevorzugt von 34,5 : 1 bis 35,5 : 1, insbesondere von 35 : 1 enthalten.

Weiterhin können Fluide den medizinischen Honig und Dexpanthenol insbesondere in einem Gewichtsverhältnis (medizinischer Honig : Dexpanthenol) von 10 : 1 bis 25 : 1, vorzugsweise von 15 : 1 bis 20 : 1, weiter vorzugsweise von 16 : 1 bis 19 : 1, besonders bevorzugt von 17 : 1 bis 18 : 1, insbesondere von 17,5 : 1 enthalten.

Fluide können zudem den medizinischen Honig und den Wirkstoffträger (insbesondere Macrogol) insbesondere in einem Gewichtsverhältnis (medizinischer Honig : Wirkstoffträger) von 10 : 1 bis 25 : 1, vorzugsweise von 15 : 1 bis 20 : 1, weiter vorzugsweise von 16 : 1 bis 19 : 1, besonders bevorzugt von 17 : 1 bis 18 : 1, insbesondere von 17,5 : 1 enthalten.

Weiterhin können Fluide Dexpanthenol und das Lokalanästhetikum (insbesondere Benzocain) insbesondere in einem Gewichtsverhältnis (Dexpanthenol : Lokalanästhetikum) von 1 : 1 bis 3 : 1, vorzugsweise von 1,8 : 1 bis 2,2 : 1, weiter vorzugsweise von 1,9 : 1 bis 2,1 : 1, besonders bevorzugt von 1,95 : 1 bis 2,05 : 1, insbesondere von 2 : 1 enthalten.

Weiterhin können Fluide den Wirkstoffträger und das Lokalanästhetikum (insbesondere Benzocain) insbesondere in einem Gewichtsverhältnis (Wirkstoffträger: Lokalanästhetikum) von 1 : 1 bis 3 : 1, vorzugsweise von 1,8 : 1 bis 2,2 : 1, weiter vorzugsweise von 1,9 : 1 bis 2,1 : 1, besonders bevorzugt von 1,95 : 1 bis 2,05 : 1, insbesondere von 2 : 1 enthalten.

Fluide können zudem Dexpanthenol und den Wirkstoffträger (insbesondere Macrogol) insbesondere in einem Gewichtsverhältnis (Dexpanthenol : Wirkstoffträger) von 0,7 : 1 bis 1,3 : 1, vorzugsweise von 0,8 : 1 bis 1,2 : 1, besonders bevorzugt von 0,9 : 1 bis 1,1 : 1, insbesondere von 1 : 1 enthalten.

Fluide, die eines oder mehrere der vorstehend angegeben Gewichtsverhältnisse (med. Honig : Lokalanästhetikum), (med. Honig : Dexpanthenol), (med. Honig : Wirkstoffträger), (Dexpanthenol : Lokalanästhetikum), (Wirkstoffträger : Lokalanästhetikum) und (Dexpanthenol : Wirkstoffträger) aufweisen, weisen eine hohe Stabilität auf und neigen nicht zur schnellen Entmischung. In einer Ausführungsform kann das Fluid aus medizinischem Honig, Lokalanästhetikum, Dexpanthenol, und Wirkstoffträger bestehen oder im Wesentlichen bestehen.

Fluide umfassen ein Lokalanästhetikum ausgewählt aus Benzocain, Lidocain, Mepivacain, Prilocain, Articain, Bupivacain, Ropivacain, Etidocain, Dyclonin, Procain, 2-Chlorprocain, Oxybuprocain, Tetracain, Formocain, Diclofenac und Gemische hiervon. Besonders bevorzugt ist, dass Fluide Benzocain und/oder Diclofenac enthalten. Besonders vorteilhaft kann ein Lokalanästhetikum sein, das eine Substanz ist oder mindestens eine Substanz umfasst, die eine örtlich begrenzte, reversible, teilweise oder vollständige Blockade der Erregungsleitung in Nervenfasern bewirken kann.

Fluide können vorzugsweise flüssiges Macrogol, insbesondere bei Raumtemperatur (z.B. bei 20°C, insbesondere bei 20°C, 1 atm.) flüssiges Macrogol, enthalten. Fluide können insbesondere Macrogol mit einer durchschnittlichen relativen molekularen Masse (average relative molecular mass) von 100 bis 600, vorzugsweise von 300 bis 500, insbesondere von 350 bis 450, weiter insbesondere von 400 enthalten. Macrogol 400 enthaltende Fluide weisen insbesondere sehr gute Eigenschaften auf. Macrogol und Verfahren zu dessen Analyse sind in European Pharmacopoeia, insbesondere in European Pharmacopoeia 6.0, Abschnitt "Macrogols", insbesondere Band 2, S. 2308 ff., beschrieben. Weiterhin können Fluide insbesondere Macrogol enthalten, das Polyethylenglykole der Formel H(-OCH₂-CH₂)ₙ-OH mit n von 4 bis 15, vorzugsweise von 8 bis 10, weiter vorzugsweise von 8 bis 9, oder von 9 bis 10, umfasst oder aus diesen besteht.

Der Wirkstoffträger kann ein pharmazeutisch akzeptabler Wirkstoffträger sein. Der Wirkstoffträger kann insbesondere ein pharmazeutischer Hilfsstoff, vorzugsweise ein organischer pharmazeutischer Hilfsstoff sein. Die Begriffe "pharmazeutischer Hilfsstoff", "Hilfsstoff für pharmazeutische Zubereitungen", und "pharmazeutisch akzeptabler Hilfsstoff" ("pharmaceutically acceptable excipient") können im Rahmen der vorliegenden Anmeldung austauschbar verwendet werden. Ein pharmazeutischer Hilfsstoff ist daher ein pharmazeutisch akzeptabler Hilfsstoff. Ein organischer pharmazeutischer Hilfsstoff kann insbesondere ein mindestens ein Kohlenstoffatom enthaltender Hilfsstoff oder ein Gemisch von Hilfsstoffen sein, die jeweils mindestens ein Kohlenstoffatom enthalten. Wirkstoffträger, insbesondere pharmazeutische Hilfsstoff(e) können vom Fachmann auf Basis seines allgemeinen Wissens und der Lehren der vorliegenden Anmeldung ausgewählt werden. Ein pharmazeutischer Hilfsstoff beziehungsweise ein pharmazeutisch akzeptabler Hilfsstoff ist ein Hilfsstoff, der zur Verabreichung an Mensch und/oder Tier geeignet ist, um eine Behandlung oder Vorbeugung einer Krankheit oder eines Zustands, insbesondere eine Wundheilung, zu erzielen ohne unangemessen schwere Nebenwirkungen unter Berücksichtigung der Schwere der Erkrankung und der Notwendigkeit der Behandlung hervorzurufen.

Der Wirkstoffträger, insbesondere der organische pharmazeutische Hilfsstoff, kann ausgewählt sein aus der Gruppe bestehend aus Füllstoff, Schmiermittel, Verdünnungsmittel, Lösungsmittel, Mittel zur Viskositätsregulation des Fluids, Diffusionsverstärker, und deren Gemischen.

Macrogol kann als Füllstoff, Schmiermittel, Lösungsmittel, Verdünnungsmittel, Mittel zur Viskositätsregulation des Fluids und Diffusionsverstärker dienen.

Der Wirkstoffträger kann ein organisches oder anorganisches Lösungsmittel, beispielsweise Wasser, oder Verdünnungsmittel sein oder umfassen. Weiterhin kann der Wirkstoffträger ein Wirkstoffträger sein, der nicht Wasser ist oder der weniger als 8 Gew.-%, vorzugsweise weniger als 3 Gew.-%, weiter vorzugsweise weniger als 2 Gew.-% an Wasser, bezogen auf das Gesamtgewicht an Wirkstoffträger, umfasst.

Falls nicht explizit abweichend angegeben, können jegliche Formen der in der vorliegenden Anmeldung angegebenen pharmazeutischen Wirk- und/oder Hilfsstoffe eingesetzt werden, beispielsweise Hydrate, Solvate und/oder Polymorphe der angegebenen Wirk- und/oder Hilfsstoffe, sowie Salze, insbesondere Säureadditionssalze der pharmazeutischen Wirk- und/oder Hilfsstoffe. Vorzugsweise werden die Wirk- und/oder Hilfsstoffe in der explizit angegebenen Form eingesetzt.

Insbesondere Fluide, die Benzocain enthalten, weisen eine überraschend gute Schmerzlinderung auf. Zudem ist von Vorteil, dass sich Benzocain gut in Dexpanthenol löst, was eine einfache und Arbeitszeit-sparende Fluidherstellung ermöglicht. Weiterhin weisen Benzocain-enthaltende Fluide eine hohe Stabilität auf und können gut gelagert werden. Darüber hinaus trägt der Wirkstoffträger, insbesondere Macrogol, zur Bildung eines Fluids mit hoher Stabilität und hoher Lagerfähigkeit bei. Überraschenderweise trägt zudem die Verdünnung des Honigs durch den Wirkstoffträger zur Verbesserung der Schmerzlinderung bei.

Das Fluid kann eine pharmazeutisch wirksame Menge an medizinischem Honig, sowie eine pharmazeutisch wirksame Menge an Lokalanästhetikum, und eine pharmazeutisch wirksame Menge an Dexpanthenol enthalten, insbesondere pharmazeutisch wirksame Mengen für eine Wundbehandlung.

Der medizinische Honig zur Herstellung des Fluids kann Manuka-Honig oder *Leptospermum* Honig enthalten; in einer Ausführungsform kann der medizinische Honig jedoch frei sein von Manuka-Honig oder *Leptospermum* Honig. Manuka-Honig kann insbesondere ein von Honig-Bienen aus dem Blütennektar der Südseemyrte (Manuka; lat. *Leptospermum scoparium*) erzeugter Honig sein.

Ein medizinischer Honig kann insbesondere ein Honig sein, der pharmazeutisch akzeptabel für ein in Kontaktbringen mit Wunden oder Haut ist. Falls erforderlich, kann der medizinische Honig steril eingesetzt werden. Falls erforderlich, kann der medizinische Honig einer Bestrahlung, einem Erhitzen, einer Filtration oder einer anderen, dem Fachmann bekannten Maßnahme zur Herstellung von medizinischem Honig unterzogen werden.

Ein Fluid gemäß der vorliegenden Erfindung weist zudem den Vorteil einer hohen Stabilität auch bei längerer Lagerung auf. Ein erfindungsgemäßes Fluid (als solches oder in Wund- oder Hautauflagen enthalten), das mit einem Kontaktbereich (insbesondere Körperoberfläche, beispielsweise Haut, Wunde) des Behandlungssubjekts (insbesondere Mensch oder Säugetier) in Kontakt gebracht ist, kann zudem eine vorteilhafte Menge an Lokalanästhetikum (insbesondere Benzocain) in einer gewünschten Zeitspanne an der Kontaktstelle des Behandlungssubjekts abgeben. Beispielsweise kann ein erfindungsgemäßes Fluid während der ersten 30 Minuten nach in Kontakt bringen mit dem Kontaktbereich bis zu 74%, des enthaltenen Lokalanästhetikums an den Kontaktbereich abgeben. Dies trägt zu einer schnellen und wirksamen Schmerzlinderung bei. Erfindungsgemäße Fluide können daher Schnellfreisetzungszusammensetzungen für Lokalanästhetika, insbesondere Benzocain, sein.

In einer Ausführungsform kann ein Fluid 0-10 Gew.-%, vorzugsweise 0-5 Gew.-%, weiter vorzugsweise 0-1 Gew.-% an Öl, insbesondere an zugesetztem Öl, enthalten oder frei von Öl, insbesondere zugesetztem Öl sein. Öl kann beispielsweise eine oder mehrere Verbindungen ausgewählt aus der Gruppe bestehend aus Estern von Alkancarbonsäuren und Alkoholen, Dialkylethern, Alkoholen, und Fettsäuretriglyceriden sein oder umfassen. In einer Ausführungsform kann das Fluid nicht in Form einer Mikroemulsion vorliegen.

In einer Ausführungsform kann das Fluid optional Wasser-in-Öl-Emulgator(en) (insbesondere zugesetzte Wasser-in-Öl-Emulgator(en)) enthalten, jedoch keine(n) Öl-in-Wasser-Emulgator(en) (insbesondere keine(n) zugesetzten Öl-in-Wasser-Emulgator(en)) enthalten. In einer Ausführungsform kann das Fluid optional Öl-in-Wasser-Emulgator(en) (insbesondere zugesetzte Öl-in-Wasser-Emulgator(en)) enthalten, jedoch keine(n) Wasser-in-Öl-Emulgator(en) (insbesondere keine(n) zugesetzte Wasser-in-Öl-Emulgator(en)) enthalten. In einer Ausführungsform kann das Fluid weniger als 5 Gew.-%, vorzugsweise weniger als 0,9 Gew.-%, vorzugsweise weniger als 0,5 Gew.-%, weiter vorzugsweise weniger als 0,3 Gew.-% an Wasser, bezogen auf die Gesamtfluidmasse, enthalten oder frei von Wasser sein. In einer Ausführungsform kann das Fluid optional Wasser-in-Öl-Emulgator(en) (insbesondere zugesetzte Wasser-in-Öl-Emulgator(en)) und Öl-in-Wasser-Emulgator(en) (insbesondere zugesetzte Öl-in-Wasser-Emulgator(en)) enthalten, wobei für den Fall, dass Wasser-in-Öl-Emulgator(en) und Öl-in-Wasser-Emulgator(en) im Fluid vorliegen, das Verhältnis an Wasser-in-Öl-Emulgator(en) (insbesondere an zugesetzten Wasser-in-Öl-Emulgator(en)) zu Öl-in-Wasser-Emulgator(en) (insbesondere an zugesetzten Öl-in-Wasser-Emulgator(en)) kleiner als 1:9, insbesondere kleiner als 1:15, oder größer als 2:1, insbesondere größer als 4:1 gewählt sein kann.

Das Fluid kann frei sein von (insbesondere zugesetztem) wasserlöslichem bei neutralem pH anionaktivem Polymer oder weniger als 0,3 Gew.-%, bezogen auf die Gesamtfluidmasse, hiervon enthalten. Zudem kann das Fluid frei sein von (insbesondere zugesetztem) wasserlöslichem bei neutralem pH kationaktivem Polymer oder weniger als 0,3 Gew.-%, bezogen auf die Gesamtfluidmasse, hiervon enthalten.

In einer Ausführungsform kann das Fluid frei von Aloe sein. In einer Ausführungsform kann das Fluid frei von Mangostanpflanzen-Derivaten, insbesondere frei von Mangostanpflanzen-Blättern, -Wurzeln, -Ästen und -Frucht oder Stücken hiervon, sowie frei von Extrakten von Mangostanpflanzen oder -früchten sein. Bei Mangostan kann es sich insbesondere um *Garcinia mangostana* L. Pflanze handeln.

Ein Fluid, das mindestens medizinischen Honig, Lokalanästhetikum, Dexpanthenol und Wirkstoffträger umfasst, kann ein Fluid zur Verwendung als Medikament sein. Weiterhin kann eine Wund- oder Hautauflage umfassend dieses Fluid eine Wund- oder Hautauflage zur Verwendung als Medikament sein.

Ein Fluid, das mindestens medizinischen Honig, Lokalanästhetikum, Dexpanthenol und Wirkstoffträger umfasst, kann ein Fluid zur Verwendung zur Behandlung und/oder Prävention einer Wunde sein, insbesondere bei Menschen und Säugetieren und Vögeln. Eine erfindungsgemäße Wund- oder Hautauflage kann eine Wund- oder Hautauflage zur Verwendung zur Behandlung und/oder Prävention einer Wunde sein, insbesondere bei Menschen und Säugetieren und Vögeln. Bei der Wunde kann es sich insbesondere um eine Wunde ausgewählt unter Ulkus (Geschwür), insbesondere Ulkus an einer Extremität, wie Bein, Fuß, Arm, Hand; Diabetes-Ulkus, Druck-Ulkus, bei Gewebstransplantation entstandene Wunde, Operationswunde, Unfallwunde, übelriechende Wunde, Ulcus cruris, traumatische Wunde, kritisch besiedelte Wunde, Verbrennung, tiefe Wunde, oberflächliche Wunde, feuchte Nekrose, akute Wunde, chronische Wunde, und infizierte (insbesondere bakteriell infizierte) Wunde handeln. Ein erfindungsgemäßes Fluid oder eine erfindungsgemäße Wund- oder Hautauflage kann auch zur Vorbeugung oder Behandlung von Infektionen, insbesondere von bakteriellen Infektionen, beispielsweise von Infektionen mit einem oder mehreren von *Pseudomonas aeruginosa*, *S. aureus*, *E. faecalis* in einer Wunde oder in an die Wunde angrenzendem Gewebe, verwendet werden.

Das Fluid oder die Wund- oder Hautauflage kann insbesondere zur topischen Anwendung eingesetzt werden. Ein erfindungsgemäßes Fluid kann zur Aufbringung im buccalen Bereich eingesetzt werden.

Gemäß einer Ausführungsform der Erfindung ist die Trägerschicht der Wund- oder Hautauflage als Netz, insbesondere Textilnetz, weiter insbesondere PET-Netz, ausgebildet. Textilnetze, insbesondere PET-Netze können das erfindungsgemäße Fluid schnell aufnehmen und ferner eine geeignete Menge speichern, so dass eine möglichst schonende Behandlung der betroffenen Hautpartie erfolgen kann. Ferner sind solche Netze kostengünstig in großen Mengen produzierbar.

Insbesondere vorteilhaft ist die Trägerschicht der erfindungsgemäßen Wund- oder Hautauflage als Vlies, insbesondere Alginatvlies, ausgebildet. Die Verwendung von Vlies, insbesondere Alginatvlies, kann die osmotische Wirkung des Honigs besonders fordern, da diese Schicht eine entsprechende gelartige Schicht im Zusammenspiel mit dem Honig bilden kann. Vlies, insbesondere Alginatvlies nimmt weiter die weiteren Additive des erfindungsgemäßen Fluids sehr gut auf und kann diese besonders schnell und hautschonende an die betreffende Hautpartie abgeben. Die Verwendung von Vlies hat die Wirkung des Lokalanästhetikums positiv beeinflusst, da somit eine schnelle Einwirkzeit erreicht wird. Das Vlies passt sich durch die Körperwärme dem menschlichen Körper sehr genau an, und somit kann das Anästhetikum lokal und gezielt auf die Nervenzellen wirken und somit entsteht die wohltuende Schmerzlinderung für den Anwender.

Die Trägerschicht kann auch als CMC(Carboxymethylcellulose)-Schicht ausgebildet sein. Diese CMC-Schicht kann gewebt oder nicht gewebt, d.h. woven oder non-woven, sein. Auch dieses Material hat sich im Zusammenhang mit dem erfindungsgemäßen Fluid als sehr vorteilhaft herausgestellt.

In einer weiteren Ausgestaltung der Erfindung ist die Trägerschicht als Viskose-Schicht ausgebildet. Viskose Schichten können ebenfalls gewebt oder nicht gewebt sein. Für die vorliegende Erfindung können alle auf Viskose Basis hergestellten Schichten Einsatz finden.

Gemäß einer Ausführungsform der vorliegenden Erfindung umfasst die Auflage eine erste Trägerschicht und eine daran anliegende zweite Trägerschicht, wobei die erste und/oder zweite Trägerschicht Fluid gemäß vorliegenden Erfindung umfassen. Diese Ausführungsform entspricht einer mehrschichtigen Ausbildung (Multilayer-Auflage) und kann im Sinne der vorliegenden Erfindung universell eingesetzt werden. So ist beispielsweise vorstellbar, dass lediglich die Schicht, die mit Haut in Kontakt kommt, Fluid aufweist, währenddessen die zweite Schicht als Schutzschicht dienen kann. Weiter können Schichten unterschiedlicher Ausbildungen verwendet werden. So kann beispielsweise die mit Haut in Kontakt kommende Schicht aus Alginatvlies bestehen und die daran angrenzende Schicht ein Textilnetz sein. Die Alginatschicht schmiegt sich genau an die Hautpartie an, und das Textilnetz (oder eine andere Alternative) dient als Schutzschicht, welche beispielsweise mit der Mullbinde in Kontakt steht.

Durch die vorliegende Erfindung kann eine nahezu schmerzfreie Behandlung von betroffenen Hautpartien gewährleistet werden.

Eine Wund- oder Hautauflage kann mindestens eine erste mit einem Fluid versetzte Trägerschicht aufweisen. Eine Trägerschicht kann unter Netz, Vlies, Textil, nichtgewebten (non-woven) Material, Flechtwerk, Strickwerk, Fasergelege, Faserbündeln, Gewirk, Filz, Schwamm, Watte, Wolle und Kombinationen hiervon ausgewählt sein. Die Trägerschicht kann ein Material umfassen oder aus diesem bestehen, das ausgewählt ist aus der Gruppe bestehend aus PET (Polyethylenterephthalat), Alginat, insbesondere Alkali-Alginat, Erdalkali-Alginat und deren Gemische, weiter insbesondere Kalzium-Alginat und Natrium-Alginat und deren Gemische, CMC (Carboxymethylcellulose), Polyester, Viskose und deren Gemischen. Eine mit einem Fluid versetzte Trägerschicht kann dafür ausgelegt sein mit einer Wunde und/oder Haut in Kontakt gebracht zu werden. In einer Ausführungsform kann die Trägerschicht vor dem Versetzen mit Fluid einen Wassergehalt von weniger als 12 Gew.-%, vorzugsweise von weniger als 5 Gew.-%, weiter vorzugsweise von weniger als 2 Gew.-%, insbesondere von weniger als 1 Gew.-% aufweisen, bezogen auf die Gesamtmasse an der mindestens einen Trägerschicht vor Versetzen mit Fluid, oder frei von Wasser sein.

Insbesondere kann eine Wund- oder Hautauflage eine mit erfindungsgemäßem Fluid beschichtete Trägerschicht umfassen, wobei die Trägerschicht beispielsweise ein Vlies, insbesondere ein Alginat-Vlies (z.B. Vlies umfassend oder bestehend aus Alkali-Alginat, Erdalkali-Alginat und deren Gemischen, insbesondere Kalzium-Alginat und Natrium-Alginat und deren Gemischen), oder ein Netz, insbesondere ein Polyesternetz, z.B. ein gestricktes Polyesternetz, sein kann oder enthalten kann.

Insbesondere können erfindungsgemäße Fluide oder Wund- oder Hautauflagen steril sein. Dies kann durch dem Fachmann bekannte Verfahren zum Sterilisieren, beispielsweise durch Einwirkung von beta-Strahlung, erreicht werden.

Überraschenderweise führt das erfindungsgemäße Fluid und die Wund- oder Hautauflage zu einer schnellen Schmerzlinderung nach Anlegen des Verbands. Bei einer Behandlung mit erfindungsgemäßen Wund- oder Hautauflagen berichteten Testpersonen von einer sehr schnellen Schmerzlinderung, die sogar noch schneller als bei Behandlung mit einer Honig-Alginat-Auflage ohne Zusatz einer Kombination von Lokalanästhetikum (insbesondere Bezocain), Dexpanthenol, und Wirkstoffträger (Macrogol, insbesondere Macrogol 400) ist. Eine detailliertere Diskussion hiervon findet sich im Beispielteil.

Eine erfindungsgemäße Wund- oder Hautauflage kann zudem Befestigungseinrichtungen aufweisen, insbesondere Befestigungsbandagen, welche eine Positionsveränderung (ein Verrutschen) der Auflage, und insbesondere der Trägerschicht, während der Verwendung verhindern oder minimieren. Alternativ oder zusätzlich kann die Wund- oder Hautauflage zudem Kompressionsbandagen aufweisen, wobei dies insbesondere bei einer Verwendung zur Behandlung von Ulcus cruris von Vorteil sein kann.

Weiterhin wird ein Verfahren zur Herstellung eines Fluids und optional zur Herstellung einer Wund-oder Hautauflage offenbart, das umfasst:
a) Herstellen einer Mischung umfassend Dexpanthenol und Lokalanästhetikum in einem ersten Temperaturbereich, (insbesondere bei einer Temperatur von mehr als 60°C,vorzugsweise bei einer Temperatur im Bereich von 65-71 °C, insbesondere bei 70°C),
b) Abkühlen der Mischung umfassend Dexpanthenol und Lokalanästhetikum auf eine Temperatur in einem zweiten Temperaturbereich, wobei der zweite Temperaturbereich außerhalb und unterhalb des ersten Temperaturbereichs liegt (insbesondere kann die Temperatur im zweiten Temperaturbereich eine Temperatur von weniger als 55°C, beispielsweise eine Temperatur von im Bereich von 47 bis 51°C, insbesondere 50°C sein),
c) Vermischen der Mischung umfassend Dexpanthenol und Lokalanästhetikum mit medizinischem Honig und einem Wirkstoffträger (insbesondere Macrogol), insbesondere mit einer Mischung von Honig und einem Wirkstoffträger (insbesondere Macrogol), bei einer Temperatur im zweiten Temperaturbereich zur Bildung eines Fluids, und
d) optional Versetzen mindestens eines Abschnitts mindestens einer Trägerschicht einer Wund- oder Hautauflage oder der gesamten mindestens einen Trägerschicht einer Wund- oder Hautauflage mit dem Fluid.

### Kurzbeschreibung der Zeichnungen

Weitere vorteilhafte Ausgestaltungen werden nachfolgend an Hand eines in der Zeichnung dargestellten Ausführungsbeispiels, sowie an Hand weiterer Beispiele, auf welche die Erfindung jedoch nicht beschränkt ist, näher beschrieben.

Es zeigen schematisch:
Fig. 1 Wundauflage gemäß einer ersten Ausführungsform der vorliegenden Erfindung; und
Fig. 2 eine weitere Ausführungsform der vorliegenden Erfindung.

### Ausführliche Beschreibung anhand eines Ausführungsbeispiels

Bei der nachfolgenden Beschreibung zweier bevorzugter Ausführungsformen der vorliegenden Erfindung bezeichnen gleiche Bezugszeichen gleiche oder vergleichbare Komponenten.

Die in der vorstehenden Beschreibung, den Ansprüchen und den Zeichnungen offenbarten Merkmale können sowohl einzeln als auch in beliebiger Kombination für die Verwirklichung der Erfindung in ihren verschiedenen Ausgestaltungen von Bedeutung sein.

### Beispiel 1 Eine Ausführungsform einer Wundauflage

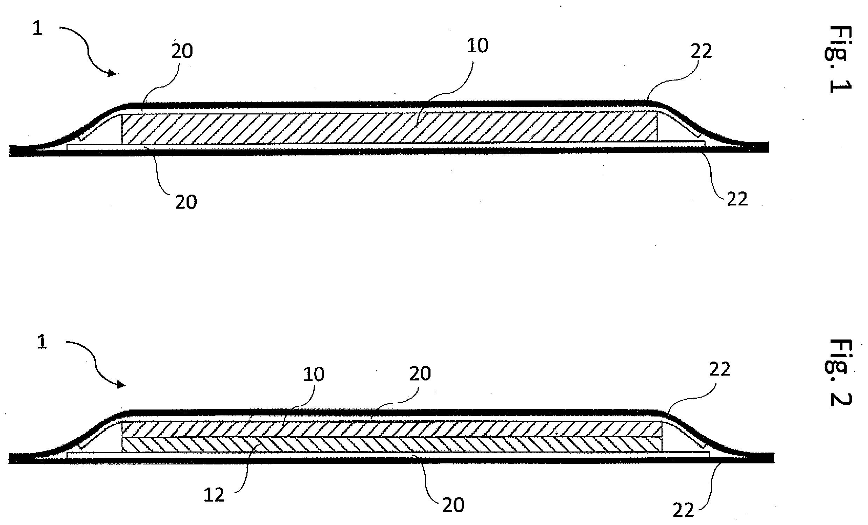

Fig. 1 zeigt eine Wundauflage gemäß einer ersten Ausführungsform der vorliegenden Erfindung. Die Auflage 1 umfasst eine erste Trägerschicht 10 für das Fluid zur Behandlung von Wunden gemäß der Erfindung. Die Trägerschicht 10 kann bevorzugt auf Alginat Basis hergestellt sein, und sie wird mit dem erfindungsgemäßen Fluid versetzt, so dass diese auf die betroffene Hautstelle eines Anwenders appliziert werden kann. Als Alginat wird bevorzugt eine Calziumalginatschicht 10 mit 50 bis 200 g/m² verwendet. Zur besseren Handhabe wird die Trägerschicht 10 zwischen zwei Trägerfolien 20 platziert, so dass ein Benutzer bei der Erstentnahme nicht mit dem Fluid, das sich auf der Trägerschicht 10 befindet, in Kontakt kommt. Durch die Verwendung dieser Folien 20 kann weiter ein Benutzer die Auflage 1 z.B. mit Hilfe einer Schere auf die gewünschte Maße bringen, so dass nur die betroffene Hautstelle mit dem Fluid in Kontakt treten kann. Der Benutzer entnimmt die Trägerschicht 10 der Auflage 1 und platziert diese auf die gewünschte Hautstelle. Als Trägerfolie 20 kann ein PET oder PP-Film mit 52 µm Dicke verwendet werden, aber auch andere Alternativen sind vorstellbar.

Schließlich umfasst die Wundauflage 1 gemäß der vorliegenden Erfindung eine Verpackungsfolie 22, die beispielsweise aus PET/ALU/PE bestehen kann. Die mit dem erfindungsgemäßen Fluid versetzte Trägerschicht 10 samt Trägerfolie 20 wird nun in der Verpackungsfolie 22 umfasst, so dass die Auflage 1 verkaufsfertig ist.

### Beispiel 2 Eine weitere Ausführungsform einer Wundauflage

Fig. 2 zeigt eine weitere Ausführungsform einer Wundauflage gemäß der vorliegenden Erfindung. Gemäß dieser Ausführungsform wird eine weitere zweite Trägerschicht 12 vorgesehen, die beispielsweise aus einem synthetischen hergestellt sein kann. So kann die zweite Trägerschicht 12 ein Textilnetz aus PET sein. Die erste und zweite Trägerschicht 10 und 12 sind flächig übereinander angeordnet und ein Benutzer kann beispielsweise entscheiden, welche Seite er auf die betroffene Hautstelle applizieren möchte.

Gemäß dieser Ausführungsform kann lediglich die erste Schicht 10, nämlich die Alginatvlies-Schicht, mit dem erfindungsgemäßen Fluid versehen sein, und die zweite Textilschicht 12 wird folglich indirekt mit dem Fluid in Kontakt kommen. So kann beispielsweise das unkontrollierte Abfließen des Fluids von der Auflage 1 kontrolliert werden.

Durch die in den Fig. 1 und 2 beschriebenen erfindungsgemäßen Wundauflagen 1, welche mit dem Fluid zur Behandlung von Wunden versetzt ist, wird folglich ein feuchtes Milieu zur Wundheilung bereitgestellt und weiter kann das verwendete Fluid eine schmerzlindernde Wirkung bei der Anwendung auf der Haut entfalten.

Mit einer klinischen Untersuchung wurde gezeigt, dass die Schmerzlinderung von Wundauflagen 1 im Sinne der vorliegenden Erfindung eine positive Wirkung auf die Wundheilung aufweist.

Gleichzeitig hat die Schmerzintensität im Wesentlichen in den ersten 30 Minuten nachgelassen, im Vergleich zu herkömmlichen Wundauflagen, welche nicht mit dem erfindungsgemäßen Fluid versehen sind. Insbesondere konnten sehr gute Resultate bei der Behandlung von chronischen Wunden beobachtet werden.

### Beispiel 3

### Fluidzusammensetzung

Ein Fluid kann folgende Zusammensetzung aufweisen:

**Tabelle 1**

| Inhaltsstoff | Gewichtsprozent, bezogen auf die Gesamtfluidmasse |
|---|---|
| medizinischer Honig | 87,5 Gew.-% |
| Lokalanästhetikum (Benzocain) | 2,5 Gew.-% |
| Dexpanthenol | 5,0 Gew.-% |
| Wirkstoffträger (Macrogol (Macrogol 400)) | 5,0 Gew.-% |

Bei 70°C wird Benzocain in Dexpanthenol verdünnt. Nach Abkühlen der Benzocain und Dexpanthenol enthaltenden Mischung auf eine Temperatur von 50°C wird eine Mischung von medizinischem Honig und Macrogol (Macrogol 400) zugegeben. Nach Mischen wird als Endprodukt ein Fluid erhalten, das 87,5 Gew.-% an medizinischem Honig, 2,5 Gew.-% an Lokalanästhetikum, 5,0 Gew.-% an Dexpanthenol, und 5,0 Gew.-% an Wirkstoffträger (Macrogol (Macrogol 400)) enthält.

Dieses Fluid weist eine überraschend hohe Stabilität, auch bei Lagerung, auf und zeigt keine Neigung zur Entmischung, insbesondere zur schnellen Entmischung der Komponenten, insbesondere zur Entmischung von Benzocain. Darüber hinaus stellt dieses Fluid eine schnelle Schmerzstillung bereit. HPLC-Tests zeigen, dass beispielsweise 74 % des Benzocains während der ersten 30 Minuten aus dem Fluid abgegeben werden können.

Dieses Fluid kann als solches verwendet werden oder die Trägerschicht einer Wund- oder Hautauflage kann mit diesem Fluid versetzt werden, wie im vorhergehenden Ausführungsbeispiel beschrieben. Falls erwünscht kann das Fluid oder die Auflage einer Sterilisierung (beispielsweise durch Einwirken von beta-Strahlung) unterzogen werden.

### Beispiel 4 Untersuchungen

Bei einer Untersuchung wurden verschiedene Typen von Wunden (insbesondere auch chronische Wunden) mit Alginat-Honig-Wund- oder Hautauflagen behandelt. Zum Vergleich wurden diese verschiedenen Typen von Wunden mit Alginat-Auflagen, Hydrocolloiden und Silber-Auflagen behandelt. Es zeigte sich bei einer Verwendung von Alginat-Honig-Wund- oder Hautauflagen eine Verringerung der für die Wundheilung chronischer Wunden benötigten Zeitdauer. Bei einer Verwendung von Alginat-Honig-Wund- oder Hautauflagen, die zusätzlich Benzocain, Macrogol und Dexpanthenol enthalten, konnte eine vergleichbar geringe Wundheilungszeitdauer beobachtet werden.

Zudem wurden Alginat-Honig-Wund- oder Hautauflagen ohne Benzocain, Macrogol und Dexpanthenol (Vergleichsvorrichtung) und Alginat-Honig-Wund- oder Hautauflagen, die erfindungsgemäßes Fluid enthalten (87,5 Gew.-% medizinischer Honig, 2,5 Gew.-% Benzocain, 5 Gew.-% Macrogol (Macrogol 400), 5 Gew.-% Dexpanthenol, bezogen auf die Fluidgesamtmasse) (erfindungsgemäße Test-Vorrichtung) miteinander verglichen:
Eine vollständige Wundheilung wurde bei 80% der mit der erfindungsgemäßen Test-Vorrichtung behandelten Personen, aber nur bei 75% der mit der Vergleichsvorrichtung behandelten Personen beobachtet. Bei einer Behandlung mit der erfindungsgemäßen Test-Vorrichtung verblieb die Umgebung der Wunde bei allen Personen intakt. Die Haut am Wundrand war jedoch weicher als bei einer Behandlung mit der Vergleichsvorrichtung. Zudem zeigt sich bei Verwendung einer erfindungsgemäßen Test-Vorrichtung eine bakteriostatische Wirkweise, insbesondere bei Wunden, die mit *Pseudomonas aeruginosa* oder *S. aureus* (*Staphylococcus aureus*) oder *E. faecalis* (*Enterococcus faecalis*) infiziert sind. Nach nur 3 bis 5 Verbandswechseln waren die Wunden gereinigt. Bei Verbandswechsel klebte die erfindungsgemäße Test-Vorrichtung nicht an der Wunde und verursachte daher keine Schmerzen während des Verbandswechsels. Bei einer Behandlung mit der erfindungsgemäßen Test-Vorrichtung wurde eine schnellere Schmerzlinderung (Schmerzlinderung bei 73% der Personen nach 30 Minuten) als bei einer Behandlung mit der Vergleichsvorrichtung (43% der Personen nach 30 Minuten) empfunden. Nach 4 Stunden waren die Schmerzen bei 87% der Personen verschwunden oder deutlich verringert. Weiterhin zeigte sich eine schnelle Wundheilung auch bei chronischen Wunden. Keine Allergien wurden beobachtet (30 Testpersonen).

### Beispiel 5 Abgabe des Lokalanästhetikums

Ein HPLC-basiertes Verfahren zur Simulation der Abgabe des Lokalanästhetikums aus einem erfindungsgemäßen Fluid (insbesondere einem Fluid mit der in Beispiel 2 angegebenen Zusammensetzung) wurde durchgeführt. Hierbei zeigte es sich, dass das Lokalanästhetikum (Benzocain) schnell freigesetzt wird und dabei zu einer guten Schmerzstillung kurz nach dem Auftragen des Verbandes beitragen kann. Dies bestätigt die Beobachtung der Testpersonen, die von einer schnellen Schmerzstillung durch das erfindungsgemäße Fluid berichten.

Das HPLC (High performance liquid chromatography)-basierte Simulationsverfahren ergibt folgendes Freisetzungsprofil für das Lokalanästhetikum (Benzocain):

**Tabelle 2**

| Zeit (min) | Gesamt (mg/cm²) |
|---|---|
| 10 | 0,825 |
| 30 | 1,0100 |
| 60 | 1,0645 |
| 120 | 1,2343 |
| 180 | 1,3156 |
| 300 | 1,3628 |

Wie aus der vorstehenden Tabelle ersichtlich, sind bereits nach 30 Minuten 74 % des Lokalanästhetikums (Benzocain) pro cm² aus der untersuchten Mischung freigesetzt. Das erfindungsgemäße Fluid zeigt daher ein Freisetzungsprofil, das in sehr vorteilhafter Weise zur Schmerzstillung nach Verbandswechseln oder beim erstmaligen Aufbringen einer Auflage auf eine Wunde oder die Haut beiträgt. Dies ist in hohem Maße von Vorteil, da Verbandswechsel bei tiefen oder großflächigen Wunden oftmals mit Schmerzen verbunden sein können.

Durch eine erfindungsgemäße Wundauflage 1 wird vorteilhaft ein feuchtes Milieu zur Wundheilung bereitgestellt, wobei das verwendete erfindungsgemäße Fluid eine schmerzlindernde Wirkung bei der Anwendung auf der Haut entfaltet.

### Bezugszeichenliste

- 1: Wund- oder Hautauflage
- 10: erste Trägerschicht, z.B Kalzium-Alginatvlies-Schicht
- 12: zweite Trägerschicht, z.B. Textilnetz
- 20: Trägerfolie
- 22: Verpackungsfolie

## Patentansprüche

1. Fluid zur Behandlung von Wunden, mindestens umfassend:
- medizinischer Honig,
- ein Lokalanästhetikum,
- Dexpanthenol und
- ein Wirkstoffträger,
wobei medizinischer Honig zu einem Anteil von 87 Gew.-% bis 88 Gew.-%, bezogen auf die Gesamtfluidmasse umfasst ist,
Lokalanästhetikum zu einem Anteil von 2 Gew.-% bis 3 Gew.-%, bezogen auf die Gesamtfluidmasse umfasst ist,
Dexpanthenol zu einem Anteil von 2 Gew.-% bis 7 Gew.-%, bezogen auf die Gesamtfluidmasse umfasst ist, und
das Lokalanästhetikum ausgewählt ist aus Benzocain, Lidocain, Mepivacain, Prilocain, Articain, Bupivacain, Ropivacain, Etidocain, Dyclonin, Procain, 2-Chlorprocain, Oxybuprocain, Tetracain, Formocain, Diclofenac und Gemischen hiervon.

2. Fluid zur Behandlung von Wunden nach Anspruch 1, **dadurch gekennzeichnet, dass** der medizinische Honig durch einen auf Kastanien basierenden Honig gebildet ist.

3. Fluid zur Behandlung von Wunden nach Anspruch 1 oder 2, **dadurch gekennzeichnet, dass** das Lokalanästhetikum Benzocain ist.

4. Fluid zur Behandlung von Wunden nach mindestens einem der vorhergehenden Ansprüche, **dadurch gekennzeichnet, dass** medizinischer Honig zu einem Anteil von 87,3 Gew.-% bis 87,6 Gew.-%, bevorzugt 87,5 Gew.-%, bezogen auf die Gesamtfluidmasse umfasst ist.

5. Fluid zur Behandlung von Wunden nach mindestens einem der vorhergehenden Ansprüche, **dadurch gekennzeichnet, dass** Lokalanästhetikum zu einem Anteil von 2,4 Gew.-% bis 2,6 Gew.-%, bevorzugt 2,5 Gew.-%, bezogen auf die Gesamtfluidmasse umfasst ist.

6. Fluid zur Behandlung von Wunden nach mindestens einem der vorhergehenden Ansprüche, **dadurch gekennzeichnet, dass** Dexpanthenol zu einem Anteil von 4,5 Gew.-% bis 5,5 Gew.-%, bevorzugt 5 Gew.-%, bezogen auf die Gesamtfluidmasse umfasst ist.

7. Fluid zur Behandlung von Wunden nach mindestens einem der vorhergehenden Ansprüche, **dadurch gekennzeichnet, dass** der Wirkstoffträger zu einem Anteil von 4 Gew.-% bis 6 Gew.-%, insbesondere 4,9 Gew.-% bis 5,1 Gew.-%, bevorzugt 5 Gew.-%, bezogen auf die Gesamtfluidmasse umfasst ist.

8. Fluid zur Behandlung von Wunden nach mindestens einem der vorhergehenden Ansprüche, **dadurch gekennzeichnet, dass** der Wirkstoffträger Macrogol ist.

9. Wund- oder Hautauflage (1), umfassend:
- mindestens eine erste mit einem Fluid nach mindestens einem der vorhergehenden Ansprüche versetzte Trägerschicht (10).

10. Wund- oder Hautauflage (1) nach Anspruch 9, **dadurch gekennzeichnet, dass** die Trägerschicht (10) als Textilnetz, insbesondere PET-Netz, ausgebildet ist.

11. Wund- oder Hautauflage (1) nach einem der Ansprüche 9 oder 10, **dadurch gekennzeichnet, dass** die Trägerschicht (10) als Vlies, insbesondere Alginatvlies, ausgebildet ist.

12. Wund- oder Hautauflage (1) nach einem der Ansprüche 9 bis 11, **dadurch gekennzeichnet, dass** die Trägerschicht (10) als CMC(Carboxymethylcellulose)-Schicht ausgebildet ist.

13. Wund- oder Hautauflage (1) nach einem der Ansprüche 9 bis 12, **dadurch gekennzeichnet, dass** die Trägerschicht (10) als Viskose-Schicht ausgebildet ist.

14. Wund- oder Hautauflage (1) nach einem der vorhergehenden Ansprüche 9 bis 13, **dadurch gekennzeichnet, dass** die Auflage (1) eine erste Trägerschicht (10) und eine daran anliegende zweite Trägerschicht (12) umfasst, wobei die erste und/oder zweite Trägerschicht (10, 12) Fluid nach mindestens einem der Ansprüche 1 bis 8 umfassen.

15. Auflage (1) nach einem der vorstehenden Ansprüche 9 bis 14 zur Verwendung bei einer feuchten Wundbehandlung.

## Claims

1. Fluid for the treatment of wounds, at least comprising:
- medical-grade honey
- a local anaesthetic
- dexpanthenol and
- an active-ingredient carrier,
wherein medical-grade honey is comprised to an extent of from 87% by weight to 88% by weight, based on the total fluid mass,
local anaesthetic is comprised to an extent of from 2% by weight to 3% by weight, based on the total fluid mass,
dexpanthenol is comprised to an extent of from 2% by weight to 7% by weight, based on the total fluid mass, and
the local anaesthetic is selected from benzocaine, lidocaine, mepivacaine, prilocaine, articaine, bupivacaine, ropivacaine, etidocaine, dyclonine, procaine, 2-chloroprocaine, oxybuprocaine, tetracaine, formocaine, diclofenac and mixtures thereof.

2. Fluid for the treatment of wounds according to Claim 1, **characterized in that** the medical-grade honey is formed by a Castanea-based honey.

3. Fluid for the treatment of wounds according to Claim 1 or 2, **characterized in that** the local anaesthetic is benzocaine.

4. Fluid for the treatment of wounds according to at least one of the preceding claims, **characterized in that** medical-grade honey is comprised to an extent of from 87.3% by weight to 87.6% by weight, preferably 87.5% by weight, based on the total fluid mass.

5. Fluid for the treatment of wounds according to at least one of the preceding claims, **characterized in that** local anaesthetic is comprised to an extent of from 2.4% by weight to 2.6% by weight, preferably 2.5% by weight, based on the total fluid mass.

6. Fluid for the treatment of wounds according to at least one of the preceding claims, **characterized in that** dexpanthenol is comprised to an extent of from 4.5% by weight to 5.5% by weight, preferably 5% by weight, based on the total fluid mass.

7. Fluid for the treatment of wounds according to at least one of the preceding claims, **characterized in that** the active-ingredient carrier is comprised to an extent of from 4% by weight to 6% by weight, particularly 4.9% by weight to 5.1% by weight, preferably 5% by weight, based on the total fluid mass.

8. Fluid for the treatment of wounds according to at least one of the preceding claims, **characterized in that** the active-ingredient carrier is macrogol.

9. Wound or skin dressing (1) comprising:
- at least one first support layer (10) admixed with a fluid according to at least one of the preceding claims.

10. Wound or skin dressing (1) according to Claim 9, **characterized in that** the support layer (10) is in the form of a textile mesh, more particularly PET mesh.

11. Wound or skin dressing (1) according to either of Claims 9 and 10, **characterized in that** the support layer (10) is in the form of a non-woven, more particularly alginate non-woven.

12. Wound or skin dressing (1) according to any of Claims 9 to 11, **characterized in that** the support layer (10) is in the form of a CMC (carboxymethylcellulose) layer.

13. Wound or skin dressing (1) according to any of Claims 9 to 12, **characterized in that** the support layer (10) is in the form of a viscose layer.

14. Wound or skin dressing (1) according to any of preceding Claims 9 to 13, **characterized in that** the dressing (1) comprises a first support layer (10) and a second support layer (12) resting thereon, wherein the first and/or second support layer (10, 12) comprise fluid according to at least one of Claims 1 to 8.

15. Dressing (1) according to any of preceding Claims 9 to 14 for use in a moist wound treatment.

## Revendications

1. Fluide pour le traitement de plaies, comprenant au moins :
- du miel médical,
- un anesthésique local,
- du dexpanthénol et
- un support de principes actifs,
le miel médical étant compris en une proportion de 87 % en poids à 88 % en poids, par rapport à la masse fluide totale,
l'anesthésique local étant compris en une proportion de 2 % en poids à 3 % en poids, par rapport à la masse fluide totale,
le dexpanthénol étant compris en une proportion de 2 % en poids à 7 % en poids, par rapport à la masse fluide totale, et
l'anesthésique local étant choisi parmi la benzocaïne, la lidocaïne, la mépivacaïne, la prilocaïne, l'articaïne, la bupivacaïne, la ropivacaïne, l'étidocaïne, la dyclonine, la procaïne, la 2-chloroprocaïne, l'oxybuprocaïne, la tétracaïne, la formocaïne, le diclofénac et leurs mélanges.

2. Fluide pour le traitement de plaies selon la revendication 1, **caractérisé en ce que** le miel médical est formé par un miel à base de châtaigniers.

3. Fluide pour le traitement de plaies selon la revendication 1 ou 2, **caractérisé en ce que** l'anesthésique local est la benzocaïne.

4. Fluide pour le traitement de plaies selon au moins l'une quelconque des revendications précédentes, **caractérisé en ce que** le miel médical est compris en une proportion de 87,3 % en poids à 87,6 % en poids, de préférence de 87,5 % en poids, par rapport à la masse de fluide totale.

5. Fluide pour le traitement de plaies selon au moins l'une quelconque des revendications précédentes, **caractérisé en ce que** l'anesthésique local est compris en une proportion de 2,4 % en poids à 2,6 % en poids, de préférence de 2,5 % en poids, par rapport à la masse de fluide totale.

6. Fluide pour le traitement de plaies selon au moins l'une quelconque des revendications précédentes, **caractérisé en ce que** la dexpanthénol est compris en une proportion de 4,5 % en poids à 5,5 % en poids, de préférence de 5 % en poids, par rapport à la masse de fluide totale.

7. Fluide pour le traitement de plaies selon au moins l'une quelconque des revendications précédentes, **caractérisé en ce que** le support de principes actifs est compris en une proportion de 4 % en poids à 6 % en poids, notamment de 4,9 % en poids à 5,1 % en poids, de préférence de 5 % en poids, par rapport à la masse de fluide totale.

8. Fluide pour le traitement de plaies selon au moins l'une quelconque des revendications précédentes, **caractérisé en ce que** le support de principes actifs est le macrogol.

9. Pansement pour les plaies ou la peau (1), comprenant :
- au moins une première couche support (10) imprégnée avec un fluide selon au moins l'une quelconque des revendications précédentes.

10. Pansement pour les plaies ou la peau (1) selon la revendication 9, **caractérisé en ce que** la couche support (10) est configurée sous la forme d'un réseau textile, notamment d'un réseau de PET.

11. Pansement pour les plaies ou la peau (1) selon l'une quelconque des revendications 9 ou 10, **caractérisé en ce que** la couche support (10) est configurée sous la forme d'un non-tissé, notamment d'un non-tissé d'alginate.

12. Pansement pour les plaies ou la peau (1) selon l'une quelconque des revendications 9 à 11, **caractérisé en ce que** la couche support (10) est configurée sous la forme d'une couche de CMC (carboxyméthylcellulose).

13. Pansement pour les plaies ou la peau (1) selon l'une quelconque des revendications 9 à 12, **caractérisé en ce que** la couche support (10) est configurée sous la forme d'une couche de viscose.

14. Pansement pour les plaies ou la peau (1) selon l'une quelconque des revendications 9 à 13 précédentes, **caractérisé en ce que** le pansement (1) comprend une première couche support (10) et une deuxième couche support (12) adjacente à celle-ci, la première et/ou la deuxième couche support (10, 12) comprenant un fluide selon au moins l'une quelconque des revendications 1 à 8.

15. Pansement (1) selon l'une quelconque des revendications 9 à 14 précédentes, destiné à une utilisation lors d'un traitement de plaies à l'état humide.
